(19) **Europäisches Patentamt**

**European Patent Office**

**Office européen des brevets**

(11) **EP 1 385 003 B1**

(12) **EUROPEAN PATENT SPECIFICATION**

(45) Date of publication and mention
of the grant of the patent:
**22.11.2006 Bulletin 2006/47**

(51) Int Cl.:
***G01N 33/564*** (2006.01)

(21) Application number: **03447196.1**

(22) Date of filing: **24.07.2003**

(54) **Reagent kit for detecting lupus anticoagulunt**

Reagenzien-Kit zum Nachweis von Lupus-Antikoagulant

Nécessaire pour la détection d'anticoagulant de Lupus

(84) Designated Contracting States:
**DE FR GB IT SE**

(30) Priority: **25.07.2002 JP 2002216782**

(43) Date of publication of application:
**28.01.2004 Bulletin 2004/05**

(73) Proprietor: **SYSMEX CORPORATION**
**Kobe-shi,**
**Hyogo 651-0073 (JP)**

(72) Inventor: **Okuda, Masahiro**
**Kobe-shi,**
**Hyogo 651-2114 (JP)**

(74) Representative: **De Clercq, Ann G. Y. et al**
**De Clercq, Brants & Partners c.v.,**
**Edgard Gevaertdreef 10a**
**9830 Sint-Martens-Latem (BE)**

(56) References cited:
**EP-A- 0 566 333**

- **KELSEY P R ET AL: "The diagnosis of lupus anticoagulants by the activated partial thromboplastin time - The central role of phosphatidyl serine" THROMBOSIS AND HAEMOSTASIS 1984 GERMANY, vol. 52, no. 2, 1984, pages 172-175, XP008024549**
- **JACOBSEN EVA M ET AL: "The evaluation of clotting times in the laboratory detection of lupus anticoagulants" THROMBOSIS RESEARCH, vol. 104, no. 4, 15 November 2001 (2001-11-15), pages 275-282, XP002261843 ISSN: 0049-3848**
- **SLATER P J ET AL: "PRO COAGULANT ACTIVITY OF PARTIAL THROMBOPLASTIN CEPHALIN REAGENT AND ITS PHOSPHO LIPID COMPOSITION" THROMBOSIS RESEARCH, vol. 18, no. 6, 1980, pages 831-838, XP008024695 ISSN: 0049-3848**
- **BRITISH JOURNAL OF HAEMATOLOGY, vol. 106, 1999, pages 801-808,**

Remarks:
The file contains technical information submitted after the application was filed and not included in this specification

EP 1 385 003 B1

**Description**

BACKGROUND OF THE INVENTION

Field of the Invention

The field of the invention relates to a blood coagulation reagent kit used in diagnosis of anti-phospholipid (aPL) antibody syndrome in the fields such as a clinical chemistry and a medical study, and more particularly pertains to a reagent kit capable of specifically detecting lupus-anticoagulant-positive diseases based on a blood test in vitro.

Discussion of the Related Art

Lupus anticoagulant (hereinafter, called as "LA") is an immunoglobulin which has been found firstly in patients suffering from systemic lupus erythematosus (hereinafter, called as "SLE patients"). While LA is detected in SLE patients with a frequency ranging from 5 to 10%, it is also detected in patients suffering from other autoimmune diseases and tumorous diseases. LA is most frequently detected in patients with anti-phospholipid syndrome (APS) such as patients with thrombosis, women who have undergone miscarriage or premature labor, and patients with thrombocytopenia. Since a time required in blood coagulation with respect to patients with APS is prolonged owing to phospholipid-dependent coagulation reaction, it is conceived that LA is an auto-antibody against phospholipid.

According to a recent study, it has been elucidated that LA is a heterogeneous immunoglobulin which shows antibody reaction against a complex of negatively-charged phospholipid and $\beta$ 2-glycoprotein I ($\beta$ 2-GPI) or a complex of negatively-charged phospholipid and prothrombin. It is conceived that LA causes anti-coagulating action because bonding of LA to the complex hinders conversion of prothrombin to thrombin.

There have been known reagents containing a certain amount of phospholipid for detecting LA having the aforementioned properties such as cephalin derived from rabbit brain, cephalin derived from bovine brain, soy bean lecithin, and viper venom. If LA exists in a blood sample, the existing LA neutralizes the phospholipid in the reagent, which gives rise to shortage of the phospholipid necessary for initiating cascade reaction of coagulating the blood. As a result of such shortage, the coagulation time of each of the blood samples is prolonged in proportion to the amount of phospholipid in the reagent which has been neutralized by LA. Thus, this approach makes it possible to determine whether each blood sample is LA positive or not.

In addition to the above approach, there has been conducted another approach in which two or more different steps are combined each other in order to secure detection accuracy, including diluting the reagent in light of the fact that the less the phospholipid in the reagent, the higher the detection sensitivity of LA is.

As an example of the latter approach, there is known a technique of judging whether a blood sample is LA-positive or not by using an activated partial thromboplastin time reagent (hereinafter, simply called as "APTT reagent"), which is a combination of two kinds of reagents having different concentrations of phospholipid from each other, and by calculating Rosner Index or Lupus Ratio (LR) based on the coagulation times respectively obtained with use of the two kinds of reagents.

One of the examples of commercially available detecting reagents is Gradipore LA (manufactured by Gradipore Ltd., Australia), which is a reagent kit of measuring coagulation times with use of Russel's viper venom. The reagent kit is a combination of a first coagulation time reagent containing viper venom and a second coagulation time reagent containing viper venom and soy-bean-derived phospholipid in an excessive amount. With use of this reagent kit, a blood sample is judged to be LA positive if the following formula is satisfied:

the ratio of the coagulation time of a blood sample added with the first coagulation time reagent to that of a blood sample added with the second coagulation time reagent $\geqq$ 1.3.

Further, according to a reported scientific research (see V.Chantarangkul, et al., Thromb. Res. 1992, 67 : 355-365; E. Rosner, et al., Thromb. Haemost. 1987, 57: 144-149), there has been established a technique in which LA is arithmetically detected by utilizing a difference in concentration of phospholipid derived from rabbit brain or its equivalent.

A still another approach is proposed. According to this approach, coagulation times are measured by using an APTT reagent kit consisting of two kinds of coagulation time reagents having different phospholipid concentrations from each other or using a diluted Russel's viper venom time (dRVVT) reagent kit with use of a sample, which is a mixture of blood plasma (normal plasma) from a normal healthy individual and blood plasma (LA-containing plasma) from an LA-positive patient in mixing ratio of 1:1, and LA is detected based on a degree of correction in correcting a deviation of the coagulation time of the LA-containing plasma from the coagulation time of the normal plasma, as an index.

In the conventional approaches of measuring the degree of phospholipid-dependent coagulation, what is used as phospholipid is the one derived from natural resources, and the composition of the phospholipid is uncontrolled. Accordingly, it is highly likely that non-uniformity of natural-resource-derived phospholipid in composition or a variation in conditions of extracting the phospholipid may adversely affect the coagulation time of a blood sample with the result that the detection sensitivity of LA is fluctuated. Further, according to the conventional measurement method, it is possible to normalize the coagulation time by addition of normal blood to a blood sample from an individual having a coagulation factor deficiency because the deficient coagulation factor is supplied from the normal blood plasma. In this case, however, it is difficult to discriminate a blood sample containing a coagulation inhibitor such as warfarin or heparin from a blood sample of an LA-positive patient. Accordingly, there is likelihood that the blood samples containing these coagulation inhibitors are misjudged as LA-positive.

In view of the above, it is necessary to discriminate a prolonged coagulation time resulting from a coagulation inhibitor or a coagulation factor deficiency from a prolonged coagulation time resulting from LA in order to precisely determine disorders resulting from LA.

## SUMMARY OF THE INVENTION

An object of this invention is to provide the use of a reagent kit and a method capable of specifically and precisely detecting LA by enhancing the detection sensitivity of LA and by discriminating between a blood sample from LA-positive individual and a blood sample of an individual having a coagulation factor deficiency or a blood sample containing a coagulation inhibitor such as heparin.

As a result of extensive research and study to attain the above object, the inventor found that blood samples from LA-positive individuals show a remarkable difference in coagulation time between the cases of using two kinds of reagents having a difference solely in the content of phosphatidylserine to phospholipid in the respective reagents, in contrast to a blood sample of an individual having a coagulation factor deficiency or a blood sample containing a coagulation inhibitor such as heparin, and thus made this invention.

In this use of a reagent kit and method; the content of phosphatidylserine to the total content of the phospholipids in the first coagulation time reagent is different from the content of phosphatidylserine to the total content of the phospholipids in the second coagulation time reagent.

Specifically the present invention relates to an in vitro use of a reagent kit for discriminating between lupus anticoagulant-positive individuals, and individuals having a coagulation factor deficiency or patients undergoing anti-coagulant therapy, said kit comprising:

a first coagulation time reagent comprising a first preparatory reagent containing phospholipids including phosphatidylserine which is synthetic phosphatidylserine or phosphatidylserine of 99% or more in purity derived from natural resources, and a second preparatory reagent containing calcium ions; and

a second coagulation time reagent comprising a third preparatory reagent containing phospholipids including phosphatidylserine which is synthetic phosphatidylserine or phosphatidylserine of 99% or more in purity derived from natural resources, and a fourth preparatory reagent containing calcium ions, wherein the content of phosphatidylserine to the total content of the phospholipids in the first coagulation time reagent is different from the content of phosphatidylserine to the total content of the phospholipids in the second coagulation time reagent, characterized in that

the concentration of phosphatidylserine in the first preparatory reagent ranges from 30 $\mu$g/ml to 100 $\mu$g/ml, and the concentration of phosphatidylserine in the third preparatory reagent ranges from 2 $\mu$g/ml to 20 $\mu$g/ml.

The present invention further relates to the use of a reagent kit as mentioned above, wherein the discrimination is based on a difference between a coagulation time measured by use of the first coagulation time reagent and a coagulation time measured by use of the second coagulation time reagent.

The present invention also relates to the use of a reagent kit as mentioned above, therein each of the first preparatory reagent and the third preparatory reagent contains phosphatidylethanolamine ranging from 0.1 $\mu$g/ml to 300 $\mu$g/ml and phosphatidylcholine ranging from 2 $\mu$g/ml to 1000 $\mu$g/ml.

The present invention also relates to the use of a reagent kit as mentioned above, wherein the concentration of the phosphatidylethanolamine in each of the first and third preparatory reagents ranges from 1 $\mu$g/ml to 30 $\mu$g/ml, and the concentration of the phosphatidylcholine in each of the first and third preparatory reagents ranges from 20 $\mu$g/ml to 100 $\mu$g/ml.

The present invention also relates to the use of a reagent kit as mentioned above, wherein each of the first and the third preparatory reagents further contains an activator, which is selected from the group consisting of ellagic acid, kaolin and sellaite.

The present invention also relates to the use of a reagent kit as mentioned above, wherein each of the first and the second coagulation time reagents further contains a viper venom.

The present invention also relates to the use of a reagent kit as mentioned above, wherein the viper venom is at least one selected from the group consisting of Russel's venom, textarin venom and ecarin venom.

The present invention also relates to the use of a reagent kit as mentioned above, wherein each of the first and the second coagulation time reagents further contains a tissue factor.

The present invention further relates to an in vitro method for discriminating between lupus anticoagulant-positive individuals, and individuals having a coagulation factor deficiency or patients undergoing anti-coagulant therapy comprising:

a step of comparing a coagulation time measured by use of a first coagulation time reagent with a coagulation time measured by use of a second coagulation time reagent, wherein the first coagulation time reagent comprises a first preparatory reagent containing phospholipids including phosphatidylserine which is synthetic phosphatidylserine or phosphatidylserine of 99% or more in purity derived from natural resources, a second preparatory reagent containing calcium ions, and the second coagulation time reagent comprises a third preparatory reagent containing phospholipids including phosphatidylserine which is synthetic phosphatidyl-serine or phosphatidylserine of 99% or more in purity derived from natural resources, and a fourth preparatory reagent containing calcium ions, and wherein the content of phosphatidylserine to the total content of the phospholipids in the first coagulation time reagent is different from the content of phosphatidylserine to the total content of the phospholipids in the second coagulation time reagent, and the concentration of phosphatidylserine in the first preparatory reagent ranges from 30 $\mu$g/ml to 100 $\mu$g/ml, and the concentration of phosphatidylserine in the third preparatory reagent ranges from 2 $\mu$g/ml to 20 $\mu$g/ml; and

a step of detecting lupus anticoagulant in blood based on the result obtained from the comparing step.

The present invention also relates to the method as mentioned above, wherein each of the first and the third preparatory reagents further contains an activator, which is selected from the group consisting of ellagic acid, kaolin and sellaite.

The present invention also relates to the method as mentioned above, wherein each of the first and the second coagulation time reagents further contains a viper venom.

The present invention also relates to the method as mentioned above, wherein the viper venom is at least one selected from the group consisting of Russel's venom, textarin venom and ecarin venom.

The present invention also relates to the method as mentioned above, wherein each of the first and the second coagulation time reagents further contains a tissue factor.

## DETAILED DESCRIPTION OF THE INVENTION

The use of a reagent kit or method for discriminating between lupus anticoagulant (hereinafter, simply called as "LA") positive individuals and individuals having a coagulation factor deficiency or patients undergoing anti-coagulant therapy, of this invention essentially consists of a first coagulation time reagent containing phospholipids including phosphatidylserine, and a second coagulation time reagent containing phospholipids including phosphatidylserine, wherein the content of the phosphatidylserine to the total content of the phospholipids in the first coagulation time reagent is different from the content of the phosphatidylserine to the total content of the phospholipids in the second coagulation time reagent.

Phosphatidylserine (hereinafter sometimes called as "PS") contained in the first coagulation time reagent and in the second coagulation time reagent is a synthetic phosphatidylserine or a phosphatidylserine derived from natural resources. It is preferable to use a synthetic phosphatidylserine. In case of using a natural-resource-derived phosphatidylserine, phosphatidylserine of 99% or more in purity is used.

Preferred phospholipids contained in the first and second coagulation time reagents other than PS include phosphatidylethanolamine (hereinafter, sometimes called as "PE") and phosphatidylcholine (hereinafter, sometimes called as "PC"). These preferred other phospholipids are necessary compositions for clot formation of a sample added with the respective first and second coagulation time reagents in vitro.

Preferably, the first (second) coagulation time reagent may contain compositions other than the above compositions that cause clot formation in vitro.

Examples of the other compositions include an activator, viper venom, calcium ions, and a tissue factor. Preferably, the activator is at least one selected from the group consisting of ellagic acid, kaolin, and sellaite. The viper venom is preferably at least one selected from the group consisting of Russel's viper venom, textarin venom, and ecarin venom. The tissue factor may be one derived from rabbit brain, derived from human placenta, or a recombinant.

The above other compositions are optionally selected depending on the kind of the coagulation time reagent. For instance, in case that the first (second) coagulation time reagent includes calcium ions and an activator, coagulation times are measured based on the principle of an activated partial thromboplastin time (APTT). In case that the first (second) coagulation time reagent includes calcium ions and viper venom, coagulation times are measured based on the principle of a Russel's viper venom time (RWT). In case that the first (second) coagulation time reagent includes calcium ions and a tissue factor, coagulation times are measured based on the principle of a prothrombin time (PT).

The first (second) coagulation time reagent may contain a buffer such as HEPES and a tris buffer when need arises to do so. The concentration of the buffer solution may be in a range allowable and generally used in the field of clinical chemistry, and is determined empirically based on simplified and repeated experiments.

Specifically, the PS concentration of the first coagulation time reagent may preferably be 10 to 20 times as high as that of the second coagulation time reagent.

More specifically, the concentration of phosphatidylserine in a mixture of a sample and the first coagulation time reagent containing phospholipids including phosphatidylserine is preferably in the range from 10 to 30 $\mu$g/ml, and more preferably in the range from 15 to 20 $\mu$g/ml. On the other hand, the concentration of phosphatidylserine in a mixture of a sample and the second coagulation time reagent containing phospholipids including phosphatidylserine is preferably in the range from 1 to 7 $\mu$g/ml, and more preferably in the range from 2 to 4 $\mu$g/ml.

Alternatively, the first (second) coagulation time reagent may be a mixture of phospholipids and the other composition(s). Further alternatively, the first coagulation time reagent may consist of a first preparatory reagent and a second preparatory reagent contained in separate vessels from each other, whereas the second coagulation time reagent may consist of a third preparatory reagent and a fourth preparatory reagent contained in separate vessels from each other. In case that the first coagulation time reagent is a combination of the first and second preparatory reagents, and the second coagulation time reagent is a combination of the third and fourth preparatory reagents, the concentrations of phosphatidylserine in the first and third preparatory reagents each containing phospholipids including phosphatidyl-serine may be such that the concentration of phosphatidylserine in the first preparatory reagent is in the range from 30 to 100 $\mu$g/ml, preferably from 40 to 60 $\mu$g/ml, whereas the concentration of phosphatidylserine in the third preparatory reagent is in the range from 2 to 20 $\mu$g/ml, preferably from 6 to 10 $\mu$g/ml. It should be noted that the concentration of phosphatidylserine in the first preparatory reagent is always higher than that in the third preparatory reagent.

In case that each of the first and the third preparatory reagents contains phosphatidylethanolamine, the concentration of phosphatidylethanolamine in the first (third) preparatory reagent may be in the range from 0.1 to 300 $\mu$g/ml, preferably from 1 to 30 $\mu$g/ml, and more preferably from 3 to 15 $\mu$g/ml. In case that each of the first and the third preparatory reagents contains phosphatidylcholine, the concentration of phosphatidylcholine in the first (third) preparatory reagent may be in the range from 2 to 1,000 $\mu$g/ml, preferably from 20 to 100 $\mu$g/ml, and more preferably from 35 to 85 $\mu$g/ml.

According to this invention, in case that the inventive use of the reagent kit or method is prepared based on the principle of APTT, the coagulation time reagent kit essentially uses the first coagulation time reagent including the first preparatory reagent and the second preparatory reagent, and the second coagulation time reagent including the third preparatory reagent and the fourth preparatory reagent, wherein the first (third) preparatory reagent contains an activator and phospholipids (phosphatidylserine, phosphatidylethanolamine, and phosphatidylcholine), and the second (fourth) preparatory reagent contains calcium ions.

Further, in case that the inventive use of the reagent kit or method is based on the principle of RVVT, each of the first and second coagulation time reagents contains phospholipids (phosphatidylserine, phosphatidylethanolamine, and phosphatidylcholine), viper venom, and calcium ions.

Furthermore, in case that the inventive use of the reagent kit or method is based on the principle of PT, each of the first and second coagulation time reagents contains phospholipids (phosphatidylserine, phosphatidylethanolamine, and phosphatidylcholine), a tissue factor, and calcium ions.

The use of the LA reagent kit or method of this invention has a feature that a combination of two kinds of reagents which differ in coagulation time from each other by differentiating the content of phosphatidylserine (PS content ratio) in the first coagulation time reagent to the total content of phospholipids from the PS content ratio in the second coagulation time reagent are used. In other words, the use of the LA reagent kit or method of this invention combine the first and second coagulation time reagents having different PS content ratios in such a manner that the coagulation times by the respective reagents are distinguishably different from each other by neutralizing PS in the first coagulation time reagent by LA.

Next, described is a method of differentiating between lupus anti coagulant-positive individuals and individuals having a coagulation factor deficiency or patients undergoing anti-coagulant therapy with use of the LA. reagent kit of this invention.

First, a sample is divided into two parts, and the first coagulation time reagent containing PS in high concentration is added to one half of the sample, whereas the second coagulation time reagent containing PS in low concentration is added to the other half of the sample. In case of using the first coagulation time reagent consisting of the first and second preparatory reagents, it is possible to mix the first and second preparatory reagents together prior to adding the mixture to the sample, or alternatively add the first (second) preparatory reagent to the sample, and then the second (or first) preparatory reagent in this order. The case of using the second coagulation time reagent consisting of the third and fourth preparatory reagents is the same as the case of using the first coagulation time reagent consisting of the first and second preparatory reagents.

In case of separately adding the first and second preparatory reagents, the mixture of the sample and the preparatory reagent may be heated according to needs after adding the first (or second) preparatory reagent or before adding the second (or first) preparatory reagent. The case of separately adding the third and fourth preparatory reagents is the same as the case of separately adding the first and second preparatory reagents.

After adding the LA reagent to the sample, the coagulation time of the sample is measured. The measuring method of the coagulation time may be a known method for a skilled person in the art, and can be measured, for example, by using a known automated analyzer.

The sample used in the measurement is preferably blood plasma in place of blood itself, more preferably, blood plasma after removal of platelets, and furthermore preferably, a mixture of blood plasma from a patient and normal blood plasma or normal blood plasma after removal of platelets. Using a mixture containing normal blood plasma as a sample is advantageous in preventing prolongation of coagulation time resulting from a coagulation factor deficiency as well as improving detection sensitivity in a test of detecting phospholipid-dependent blood coagulation disorder. The mixing ratio of blood plasma from a patient to normal blood plasma generally ranges from 4:1 to 1:4, and preferably is 1:1.

Coagulation tests using the LA reagent kit of this invention show that the coagulation times of samples having anticoagulant disorders such as blood samples from LA-positive patients, patients having a blood coagulation factor deficiency, patients administered with warfarin, and patients administered with heparin are longer than the coagulation times of samples of normal blood. Further, there is recognized a difference in coagulation time between use of the first coagulation time reagent and use of the second coagulation time reagent with respect to the samples from LA-positive patients. Specifically, observation on coagulation tests with respect to the samples from LA-positive patients reveals that the coagulation time of the sample in use of the second coagulation time reagent containing low-concentrated PS is longer than that of the sample in use of the first coagulation time reagent containing high-concentrated PS. On the other hand, observation on coagulation tests with respect to the samples from patients having a blood coagulation factor deficiency, patients administered with warfarin, and patients administered with heparin reveals that there is not found a noticeable difference in coagulation time between use of the first coagulation reagent and use of the second coagulation time reagent. In view of these observations, LA is specifically detectable based on a coagulation time difference between use of the first coagulation reagent and use of the second coagulation reagent.

It is true that LA is detectable by judging a coagulation time difference between use of the first coagulation reagent and use of the second coagulation reagent. In order to diagnose a sample from a patient having LA disease as LA-positive with high reliance, judging from a ratio of coagulation time of a blood sample from a patient to coagulation time of a normal blood sample, for example Rosner Index (see E. Rosner, et al., Thromb. Haemast. 1987, 57: 144-149) or Lupus ratio (see R. Schjetlein, et al., Thromb. Res. 1993, 69: 239-250), is preferred.

In case of samples of normal blood plasma, the PS content ratio does not affect the coagulation time. Accordingly, the LR value corresponding to the ratio of the second coagulation time reagent to the first coagulation time reagent is around 1 with respect to the samples of normal blood plasma. Likewise, the LR value of each of the samples from patients having a blood coagulation factor deficiency, patients administered with warfarin, or patients administered with heparin is around 1 because there has not been recognized a great coagulation time difference due to a difference in PS concentration despite the fact that the coagulation times of these samples are longer compared to the coagulation times of the samples of normal blood. On the other hand, in case of samples of LA-positive patients, despite use of the first and second coagulation time reagents in various concentrations and combinations thereof, it is obvious that the coagulation time in use of the second coagulation time reagent is distinguishably longer than that in use of the first coagulation time reagent. Accordingly, the reagent kit of this invention is advantageous in determining LA-positive by judging whether the LR value of each of the samples is larger than a reference value.

As mentioned above, the use of the LA reagent kit or method of this invention has a feature that the concentration of PS specifically bound to LA is controllably varied between the two reagents. Accordingly, the use of the LA reagent kit or method of this invention makes it possible for medical personnel diagnosing LA-positive patients to recognize the coagulation time difference easily. On the other hand, the conventional reagent kit is a combination of reagents containing natural-resource-derived phospholipid which is uncontrolled in composition. Accordingly, the conventional reagent kit fails to discriminate LA-positive patients from individuals having other anticoagulant diseases. Use of the LA reagent kit of this invention is advantageous in exclusively determining LA-positive patients without likelihood that samples from individuals administered with an inhibitor such as heparin are erroneously judged as LA-positive.

EXAMPLES

This invention will be further illustrated below by means of a number of concrete practical examples, which however do not in any way restrict the scope of the invention.

Example

A first and a third preparatory reagents were prepared by mixing HEPES buffer, ellagic acid, TRIS buffer, phosphatidylethanolamine (PE), phosphatidylcholine (PC) and phosphatidylserine (PS). The resulting first preparatory reagent had a pH of 7.35 and respective concentrations of 50mM of HEPS buffer, 0.1mM of ellagic acid, 25mM of tris buffer, 50 $\mu$g/ ml of PE, 50 $\mu$g/ ml of PS, and 70 $\mu$g/ ml of PC. The resulting third preparatory reagent had a pH of 7.35 and respective concentrations of 50mM of HEPES buffer, 0.1mM of ellagic acid, 25mM of tris buffer, 50 $\mu$g/ ml of PE, 10 $\mu$g/ ml of PS, and 70 $\mu$ g/ ml of PC. A second and a fourth preparatory reagents each containing 25mM/ ml of calcium ions were prepared by adding $CaCl_2$ to purified water.

COAGUTROL N ® was used as a sample of normal plasma. A mixture of patient's plasma and the normal plasma in one-to-one ratio was used as a sample of abnormal blood plasma. As samples of abnormal plasma, four kinds of plasma samples from LA-positive patients, four kinds of plasma samples from patients administered with warfarin and a plasma sample from a patient administered with heparin were used.

Each of the samples prepared above was divided into two groups. Each sample of the one group was subjected to the treatment with the first coagulation time reagent (hereinafter called as "H-APTT reagent"), while each sample of the other group was subjected to the treatment with the second coagulation time reagent (hereinafter called as "L-APTT reagent"). The treatment with H-APTT reagent was conducted in a manner that a mixture of 50 $\mu$l of each sample and the above-prepared first preparatory reagent was heated for three minutes at 37°C and then 50 $\mu$l of the above-prepared second preparatory reagent was added to trigger coagulation. The treatment with L-APTT reagent was conducted in the same manner as the treatment with H-APTT reagent except for use of the third and fourth preparatory reagents instead of the first and second preparatory reagents respectively.

Coagulation time, which may be measured as a time required for a certain clot formation, was measured with an automatic blood coagulation analyzer "Coagrex- 800" (available for Shimadzu Corp.). The measurements were conducted twice and the averages of the measurements were calculated. Lupus ratio (LR) of each sample was calculated according to the formula below (described in R. Schjetlein, et al., Thromb. Res. 1993, 69: 239-250).

$$\text{Lupus Ratio(LR)} = (b / a) / (d / c) = bc / ad$$

wherein "a", "b", " c" and "d" are values measured under the conditions in Table 1.

TABLE 1

|  | H-APTT | L-APTT |
|---|---|---|
| Mixture of patient's plasma and normal plasma | a | b |
| Pool of normal plasma | c | d |

The results for blood samples are shown in Table 2.

Clearly shown in Table 2, the coagulation times of abnormal plasma samples were prolonged comparing with the coagulation times of normal plasma samples. Furthermore, results indicate that LA-positive plasma samples treated with the L-APTT reagent have longer coagulation times than ones treated with the H-APTT reagent, while other abnormal plasma samples (i.e. samples of blood administered with warfarin or heparine) treated with the L-APTT reagent have similar coagulation times to ones treated with the H-APTT reagent.

TABLE 2

| SAMPLE | Coagulation time (sec.) | | LR value | Decision |
|---|---|---|---|---|
|  | L-APTT | H-APTT |  |  |
| Normal plasma | 29.6 | 29.4 | 1.00 | - |
| Containing Heparin | 33.5 | 35.3 | 0.94 | - |
| Containing Warfarin 1 | 62.7 | 67.7 | 0.92 | - |
| Containing Warfarin 2 | 39.9 | 39.5 | 1.00 | - |
| Containing Warfarin 3 | 37.9 | 38.8 | 0.97 | - |

(continued)

| SAMPLE | Coagulation time (sec.) | | LR value | Decision |
|---|---|---|---|---|
| | L-APTT | H-APTT | | |
| Containing Warfarin 4 | 34.5 | 32.6 | 1.05 | - |
| LA positive 1 | 101.5 | 57.7 | 1.75 | + |
| LA positive 2 | 106.8 | 59.2 | 1.79 | + |
| LA positive 3 | 95.4 | 54.3 | 1.75 | + |
| LA positive 4 | 114.5 | 65.4 | 1.74 | + |

Samples were judged as positive (+) when the LR value of the sample is larger than 1.1, and judged as negative (--) when the LR value of the sample is not larger than 1.1. Clearly shown in Table 2, all samples of LA positive plasma were determined as positive(+), while the other abnormal plasma samples were determined as negative(--). Therefore, use of the inventive reagent kit is useful for easily and accurately distinguishing between blood samples from patients having disposition for LA-positive and other blood samples from individuals having disorders in blood coagulation.

Comparative example

The coagulation times of samples were measured according to LA test "Gradipore" (available for Medical Biological Laboratory). In this test, it is used the reagent kit which is a combination of a coagulation time reagent containing viper venom (hereinafter called as "RVVT reagent") and a coagulation time reagent containing viper venom and soybean-derived phospholipid in an excessive amount (hereinafter called as "diluted RVVT reagent"). As samples to be measured, the same kinds of samples as ones used in the above example were used. After measuring the coagulation times of samples, LR values were calculated in the same manner as the example. The results are shown in Table 3.

TABLE 3

| SAMPLE | Coagulation time (sec.) | | LR value | Decision |
|---|---|---|---|---|
| | d-RVVT | RVVT | | |
| Normal plasma | 36.8 | 31.4 | 1.00 | |
| Containing Heparin | 56.4 | 30.2 | 1.59 | + |
| Containing Warfarin 1 | 116.9 | 65.1 | 1.53 | + |
| Containing Warfarin 2 | 59.8 | 38.9 | 1.31 | + |
| Containing Warfarin 3 | 57.1 | 36.5 | 1.33 | + |
| Containing Warfarin 4 | 61.3 | 35.7 | 1.47 | + |
| LA positive 1 | 74.1 | 35.5 | 1.78 | + |
| LA positive 2 | 81.4 | 36.6 | 1.90 | + |
| LA positive 3 | 62.3 | 33.2 | 1.60 | + |
| LA positive 4 | 81.2 | 38.7 | 1.79 | + |

As seen in Table 3, coagulation time of every abnormal plasma sample treated with the diluted RVVT reagent was prolonged comparing with one of a normal plasma sample treated with the same reagent. However, not only LA-positive plasma samples treated with the diluted RVVT reagent but also other abnormal blood plasma samples treated with the same showed longer coagulation times than samples treated with the RVVT reagent.
Accordingly the combinations of reagents having a different content of PS to the total content of phospholipids from each other may be effective for distinguishing between blood samples from LA-positive patients and blood samples having other disorders in blood coagulation.

**Claims**

1.  In vitro use of a reagent kit for discriminating between lupus anticoagulant-positive individuals, and individuals having a coagulation factor deficiency or patients undergoing anti-coagulant therapy, said kit comprising:

    a first coagulation time reagent comprising a first preparatory reagent containing phospholipids including phosphatidylserine which is synthetic phosphatidylserine or phosphatidylserine of 99% or more in purity derived from natural resources, and a second preparatory reagent containing calcium ions; and
    a second coagulation time reagent comprising a third preparatory reagent containing phospholipids including phosphatidylserine which is synthetic phosphatidylserine or phosphatidylserine of 99% or more in purity derived from natural resources, and a fourth preparatory reagent containing calcium ions, wherein the content of phosphatidylserine to the total content of the phospholipids in the first coagulation time reagent is different from the content of phosphatidylserine to the total content of the phospholipids in the second coagulation time reagent, **characterized in that**
    the concentration of phosphatidylserine in the first preparatory reagent ranges from 30 $\mu$g/ml to 100 $\mu$g/ml, and the concentration of phosphatidylserine in the third preparatory reagent ranges from 2 $\mu$g/ml to 20 $\mu$g/ml.

2.  The use according to claim 1 wherein the discrimination is based on a difference between a coagulation time measured by use of the first coagulation time reagent and a coagulation time measured by use of the second coagulation time reagent.

3.  The use according to claim 1 or 2, wherein each of the first preparatory reagent and the third preparatory reagent contains phosphatidylethanolamine ranging from 0.1 $\mu$g/ml to 300 $\mu$g/ml and phosphatidylcholine ranging from 2 $\mu$g/ml to 1000 $\mu$g/ml.

4.  The use according to any of claims 1 to 3, wherein the concentration of the phosphatidylethanolamine in each of the first and third preparatory reagents ranges from 1 $\mu$g/ml to 30 $\mu$g/ml, and the concentration of the phosphatidylcholine in each of the first and third preparatory reagents ranges from 20 $\mu$g/ml to 100 $\mu$g/ml.

5.  The use according to any of claims 1 to 4, wherein each of the first and the third preparatory reagents further contains an activator, which is selected from the group consisting of ellagic acid, kaolin and sellaite.

6.  The use according to any of claims 1 to 5, wherein each of the first and the second coagulation time reagents further contains a viper venom.

7.  The use according to claim 6, wherein the viper venom is at least one selected from the group consisting of Russel's venom, textarin venom and ecarin venom.

8.  The use according to any of claims 1 to 7, wherein each of the first and the second coagulation time reagents further contains a tissue factor.

9.  An in vitro method for discriminating between lupus anticoagulant-positive individuals, and individuals having a coagulation factor deficiency or patients undergoing anti-coagulant therapy comprising:

    a step of comparing a coagulation time measured by use of a first coagulation time reagent with a coagulation time measured by use of a second coagulation time reagent, wherein the first coagulation time reagent comprises a first preparatory reagent containing phospholipids including phosphatidylserine which is synthetic phosphatidylserine or phosphatidylserine of 99% or more in purity derived from natural resources, a second preparatory reagent containing calcium ions, and the second coagulation time reagent comprises a third preparatory reagent containing phospholipids including phosphatidylserine which is synthetic phosphatidyl-serine or phosphatidyl-serine of 99% or more in purity derived from natural resources, and a fourth preparatory reagent containing calcium ions, and wherein the content of phosphatidylserine to the total content of the phospholipids in the first coagulation time reagent is different from the content of phosphatidylserine to the total content of the phospholipids in the second coagulation time reagent, and the concentration of phosphatidylserine in the first preparatory reagent ranges from 30 $\mu$g/ml to 100 $\mu$g/ml, and the concentration of phosphatidylserine in the third preparatory reagent ranges from 2 $\mu$g/ml to 20 $\mu$g/ml; and
    a step of detecting lupus anticoagulant in blood based on the result obtained from the comparing step.

10. The method according to claim 9, wherein each of the first and the third preparatory reagents further contains an activator, which is selected from the group consisting of ellagic acid, kaolin and sellaite.

11. The method according to claim 9 or 10, wherein each of the first and the second coagulation time reagents further contains a viper venom.

12. The method according to claim 11, wherein the viper venom is at least one selected from the group consisting of Russel's venom, textarin venom and ecarin venom.

13. The method according to any of claims 9 to 12, wherein each of the first and the second coagulation time reagents further contains a tissue factor.

**Patentansprüche**

1. In-vitro-Verwendung eines Reagenskits zur Unterscheidung zwischen Lupus-Antikoagulanz-positiven Individuen und Individuen mit einer Koagulationsfaktordefizienz oder Patienten, welche antikoagulative Therapie erhalten, welches Kit umβast:

ein erstes Koagulationszeitreagens, umfassend ein erstes Vorbereitungsreagens enthaltend Phospholipide umfassend Phosphatidylserin, welches synthetisches Phosphatidylserin oder von natürlichen Quellen erhaltenes Phosphatidylserin mit einer Reinheit von 99 % oder mehr ist, und ein zweites Vorbereitungsreagens enthaltend Calciumionen; und

ein zweites Koagulationszeitreagens, umfassend ein drittes Vorbereitungsreagens enthaltend Pospholipide umfassend Phosphatidylserin, welches synthetisches Phosphatidylserin oder von natürlichen Quellen erhaltenes Phosphatidylserin mit einer Reinheit von 99 % oder mehr ist, und ein viertes Vorbereitungsreagens enthaltend Calciumionen, worin der Gehalt an Phosphatidylserin zu dem Gesamtgehalt der Phospholipide in dem ersten Koagulationszeitreagens unterschiedlich ist zu dem Gehalt an Phosphatidylserin zu dem Gesamtgehalt der Phospholipide in dem zweiten Koagulationszeitreagens, **gekennzeichnet dadurch, daß** die Konzentration von Phosphatidylserin in dem ersten Vorbereitungsreagens im Bereich von 30 $\mu$g/ml bis 100 $\mu$g/ml liegt, und die Konzentration von Phosphatidylserin in dem dritten Vorbereitungreagens in dem Bereich von 2$\mu$g/ml bis 20 $\mu$g/ml liegt.

2. Verwendung gemäß Anspruch 1, worin die Unterscheidung sich auf einen Unterschied in der Koagulationszeit stützt, die unter Verwendung des ersten Koagulationszeitreagens gemessen wurde, und einer Koagulationszeit, die unter Verwendung des zweiten Koagulationszeitreagens gemessen wurde.

3. Verwendung gemäß Anspruch 1 oder 2, worin sowohl das erste Vorbereitungsreagens und das dritte Vorbereitungsreagens Phosphatidylethanolamin in einem Bereich von 0,1 $\mu$g/ml bis 300 $\mu$g/ml und Phosphatidylcholin in einem Bereich von 2 $\mu$g/ml bis 1.000 $\mu$g/ml enthält.

4. Verwendung gemäß einem der Ansprüche 1 bis 3, worin die Konzentration des Phosphatidylethanolamins in sowohl dem ersten als auch dem dritten Vorbereitungsreagens im Bereich von 1 $\mu$g/ml bis 30 $\mu$g/ml liegt, und die Konzentration des Phosphatidylcholins in sowohl dem ersten als auch dem dritten Vorbereitungsreagens im Bereich von 20 $\mu$g/ml bis 100 $\mu$g/ml liegt.

5. Verwendung gemäß einem der Ansprüche 1 bis 4, worin sowohl das erste als auch das dritte Vorbereitungsreagens des weiteren einen Aktivator enthält, welcher ausgewählt ist aus der Gruppe bestehend aus Ellagsäure, Kaolin und Sellait.

6. Verwendung gemäß einem der Ansprüche 1 bis 5, worin sowohl das erste als auch das zweite Koagulationszeitreagens des weiteren Schlangengift enthält.

7. Verwendung gemäß Anspruch 6, worin das Schlangengift zumindest eines ausgewählt aus der Gruppe bestehend aus dem Gift der Kettenviper ("Russel's venom"), dem Gift der Braunschlange ("textarin venom") und dem Gift der Sandrasselotter ("ecarin venom") ist.

8. Verwendung gemäß einem der Ansprüche 1 bis 7, worin sowohl das erste als auch das zweite Koagulationszeitreagens des weiteren einen Gewebefaktor enthält.

9.  In-vitro-Verfahren zur Unterscheidung zwischen Lupus-Antikoagulanzpositiven Individuen und Individuen mit einer Koagulationsfaktordefizienz oder Patienten, welche antikoagulative Therapie erhalten, umfassend:

    einen Schritt, in dem eine Koagulationszeit, die unter Verwendung des ersten Koagulationszeitreagens gemessen wurde, mit einer Koagulationszeit, die unter Verwendung eines zweiten Koagulationszeitreagens gemessen wurde, verglichen werden, worin das erste Koagulationszeitreagens ein erstes Vorbereitungsreagens umfaßt enthaltend Phospholipide umfassend Phosphatidylserin, welches synthetisches Phosphatidylserin oder Phosphatidylserin erhalten aus natürlichen Quellen mit einer Reinheit von 99 % oder mehr ist, ein zweites Vorbereitungsreagens enthaltend Calciumionen und das zweite Koagulationszeitreagens, umfassend ein drittes Vorbereitungsreagens enthaltend Phospholipide umfassend Phosphatidylserin, welches synthetisches Phosphatidylserin oder Phosphatidylserin erhalten aus natürlichen Quellen mit einer Reinheit von 99 % oder mehr ist, und ein viertes Vorbereitungsreagens enthaltend Calciumionen, und worin der Gehalt von Phosphatidylserin zu dem Gesamtgehalt der Phospholipide in dem ersten Koagulationszeitreagens unterschiedlich ist von dem Gehalt an Phosphatidylserin zu dem Gesamtgehalt von Phospholipiden in dem zweiten Koagulationszeitreagens, und die Konzentration von Phosphatidylserin in dem ersten Vorbereitungsreagens im Bereich von 30 $\mu$g/ml bis 100 $\mu$g/ml liegt, und die Konzentration von Phosphatidylserin in dem dritten Vorbereitungsreagens im Bereich von 2 $\mu$g/ml bis 20 $\mu$g/ml liegt; und
    einen Schritt, Lupus-Antikoagulanz im Blut zu detektieren auf Basis des in dem Vergleichsschritt erhaltenen Resultats.

10. Verfahren gemäß Anspruch 9, worin sowohl das erste als auch das dritte Vorbereitungsreagens des weiteren einen Aktivator enthält, welcher ausgewählt ist aus der Gruppe bestehend aus Ellagsäure, Kaolin und Sellait.

11. Verfahren gemäß Anspruch 9 oder 10, worin sowohl das erste als auch das zweite Koagulationszeitreagens des weiteren ein Schlangengift enthält.

12. Verfahren gemäß Anspruch 11, worin das Schlangengift zumindest eines ausgewählt aus der Gruppe bestehend aus dem Gift der Kettenviper ("Russel's venom"), dem Gift der Braunschlange ("textarin venom") und dem Gift der Sandrasselotter ("ecarin venom") ist.

13. Verfahren gemäß einem der Ansprüche 9 bis 12, worin sowohl das erste als auch das zweite Koagulationszeitsreagens des weiteren einen Gewebefaktor enthält.

## Revendications

1.  Utilisation in vitro d'un kit de réactifs pour distinguer des individus souffrant d'un lupus anticoagulant positif et des individus ayant un facteur de coagulation déficient ou des patients recevant une thérapie à base d'anticoagulant, ledit kit comprenant :

    - un premier réactif de temps de coagulation comprenant un premier réactif de préparation contenant des phospholipides incluant la phosphatidylsérine laquelle est la phosphatidylsérine synthétique ou la phosphatidylsérine purifiée à 99% ou plus, dérivée de ressources naturelles, et un second réactif de préparation contenant des ions calcium ; et
    - un second réactif de temps de coagulation comprenant un troisième réactif de préparation contenant des phospholipides incluant la phosphatidylsérine laquelle est la phosphatidylsérine synthétique ou la phosphatidylsérine purifiée à 99% ou plus, dérivée de ressources naturelles, et un quatrième réactif de préparation contenant des ions calcium, dans lequel le contenu en phosphatidylsérine par rapport au contenu total des phospholipides dans le premier réactif de temps de coagulation est différent du contenu en phosphatidylsérine par rapport au contenu total des phospholipides dans le second réactif de temps de coagulation, **caractérisé en ce que**

    la concentration en phosphatidylsérine dans le premier réactif de préparation s'étend de 30 $\mu$g/ml à 100 $\mu$g/ml, et la concentration en phosphatidylsérine dans le troisième réactif de préparation s'étend de 2 $\mu$g/ml à 20 $\mu$g/ml.

2.  Utilisation selon la revendication 1, dans laquelle la distinction est basée sur une différence entre un temps de coagulation mesuré par l'utilisation du premier réactif de temps de coagulation et un temps de coagulation mesuré par l'utilisation du second réactif de temps de coagulation.

3. Utilisation selon les revendications 1 ou 2, dans laquelle le premier réactif de préparation et le troisième réactif de préparation contiennent chacun de la phosphatidyléthanolamine dans la gamme de 0,1 μg/ml à 300 μg/ml et de la phosphatidylcholine dans la gamme de 2 μg/ml à 1000 μg/ml.

4. Utilisation selon l'une quelconque des revendications 1 à 3, dans laquelle la concentration de la phosphatidyléthanolamine dans le premier et le troisième réactif de préparation s'étend pour chacun de 1 μg/ml à 30 μg/ml, et la concentration de la phosphatidylcholine dans le premier et le troisième réactif de préparation s'étend pour chacun de 20 μg/ml à 100 μg/ml.

5. Utilisation selon l'une quelconque des revendications 1 à 4, dans laquelle le premier et le troisième réactif de préparation contiennent chacun en plus un activateur, lequel est sélectionné dans le groupe constitué d'acide ellagique, de kaolin et de sellaïte.

6. Utilisation selon l'une quelconque des revendications 1 à 5, dans laquelle le premier et le second réactif de temps de coagulation contiennent chacun en plus du venin de vipère.

7. Utilisation selon la revendication 6, dans laquelle le venin de vipère est au moins sélectionné dans le groupe constituant le venin de Russell, le venin de textarine et le venin d'écarine.

8. Utilisation selon l'une quelconque des revendications 1 à 7, dans laquelle le premier et le second réactif de temps de coagulation contiennent chacun en plus un facteur tissulaire.

9. Méthode in vitro pour distinguer des individus souffrant d'un lupus anticoagulant positif et des individus ayant un facteur de coagulation déficient ou des patients recevant une thérapie à base d'anticoagulant, comprenant :

   - une étape de comparaison du temps de coagulation mesuré par l'utilisation d'un premier réactif de temps de coagulation avec un temps de coagulation mesuré par l'utilisation d'un second réactif de temps de coagulation, dans laquelle le premier réactif de temps de coagulation comprend un premier réactif de préparation contenant des phospholipides incluant la phosphatidylsérine laquelle est la phosphatidylsérine synthétique ou la phosphatidylsérine purifiée à 99% ou plus, dérivée de ressources naturelles, un second réactif de préparation contenant des ions calcium, et le second réactif de temps de coagulation comprend un troisième réactif de préparation contenant des phospholipides incluant de la phosphatidylsérine laquelle est la phosphatidylsérine synthétique ou la phosphatidylsérine purifiée à 99% ou plus, dérivée de ressources naturelles, et un quatrième réactif de préparation contenant des ions calcium, et dans lequel le contenu en phosphatidylsérine par rapport au contenu total des phospholipides dans le premier réactif de temps de coagulation est différent du contenu en phosphatidylsérine par rapport au contenu total des phospholipides dans le second réactif de temps de coagulation, et la concentration en phosphatidylsérine dans le premier réactif de préparation s'étend de 30 μg/ml à 100 μg/ml, et la concentration en phosphatidylsérine dans le troisième réactif de préparation s'étend de 2 μg/ml à 20 μg/ml ; et
   - une étape de détection de lupus anticoagulant dans le sang, basée sur le résultat obtenu dans l'étape de comparaison.

10. Méthode selon la revendication 9, dans laquelle le premier et troisième réactif contiennent chacun en plus un activateur, lequel est sélectionné dans le groupe constitué de d'acide ellagique, de kaolin et de sellaïte.

11. Méthode selon les revendications 9 ou 10, dans laquelle le premier et le second réactif de temps de coagulation contiennent chacun en plus du venin de vipère.

12. Méthode selon la revendication 11, dans laquelle le venin de vipère est au moins sélectionné dans le groupe constituant le venin de Russel, le venin de textarine et le venin d'écarine.

13. Méthode selon l'une quelconque des revendications 9 à 12, dans laquelle le premier et le second réactif de temps de coagulation contiennent chacun en plus un facteur tissulaire.